# EUROPEAN PATENT SPECIFICATION

(11) **EP 1 891 418 B1**
(45) Date of publication and mention of the grant of the patent: **18.01.2017**
(21) Application number: 06799950.8
(22) Date of filing: 19.06.2006
(51) Int. Cl.: G01N 33/14, C11D 11/00, G01N 21/64, G01N 21/84

(54) **FLUOROMETRIC METHOD FOR MONITORING A CLEAN-IN-PLACE SYSTEM**
FLUOROMETRISCHES VERFAHREN ZUR ÜBERWACHUNG EINES IN-SITU-REINIGUNGSSYSTEMS
METHODE FLUOROMETRIQUE DE SURVEILLANCE DE SYSTEME DE NETTOYAGE EN PLACE

(30) Priority: 17.06.2005 US 155286
(43) Date of publication of application: 27.02.2008
(73) Proprietor: Nalco Company, Naperville, IL 60563-1198 (US)
(72) Inventor: HOTSLANDER, John, La Crosse, WI 54601 (US); HOOTS, John, St. Charles, IL 60174 (US); VAN CAMP, James, R., Glen Ellyn, IL 60137 (US)
(74) Representative: Godemeyer Blum Lenze Patentanwälte Partnerschaft mbB - werkpatent
(86) International application number: PCT/US2006/023700
(87) International publication number: WO 2006/135938

(56) References cited:
- WO-A1-94/09357
- US-A- 4 783 314
- US-A- 5 441 611
- US-A- 5 702 684
- US-A1- 2004 132 195
- US-A1- 2004 132 195

## Description

### FIELD OF THE INVENTION

The invention pertains to a method for fluorometrically monitoring a Clean-In-Place ("CIP") system and for fluorometrically monitoring the dosage of chemical added to the CIP system.

### BACKGROUND OF THE INVENTION

Beverages and similar liquid pumpable food products are prepared, processed and package in bottles, cans, flexible or other containers through fillers connected to piping, tankage and so forth. Whenever a new type of beverage, etc. is to be filled, all of the product-contacting surfaces must be cleaned of any residues from the previous product in that system. A common occurrence in a non-alcoholic beverage "bottling" plant would be the change from a "Diet" formula to one containing sugars and other nutritional ingredients. Simultaneously there are sterilization procedures that must be used to adhere to FDA and other regulatory agencies to assure food hygiene and safety.

Some facilities carry out this task without having to dismantle and clean individual parts. This process is known as a CIP system. In particular some facilities use a thermal process, often referred to as a 3-Step CIP system, which requires temperatures capable of pasteurizing the system while removing residues of food and impurities.

The 3-step CIP system, as generally carried out, involves a sequence of separate operations: (1) Pre-Rinse; (2) Clean; and (3) Final Rinse. The sequence proceeds as follows:
1. PRE-RINSE - Following the filling of the last container of the particular flavor the determination is made by the production scheduler that a change in product, or the cessation of packaging for a work-day is at hand. An initial rinse begins the CIP system by flushing out residues of the beverage product with clean water.
2. CLEAN - After a specified time period, the process piping system is connected to a dedicated tank into which the sanitizing and cleaning chemicals are injected according to GMP for the facility. The cleaning step may involve an alkaline or acid wash. For a thermal disinfection, the water is recirculated through a heat exchanger to raise the temperature to approximately 85°C (185°F), and then is maintained at that temperature throughout the cleaning cycle. For cleaning only, the cleaner solution is circulated with or without heating to dislodge any residues or contaminants from the surfaces of the system. After the specified period has passed the Clean step is complete.
3. FINAL RINSE -While continuing to recirculate the water through the system, fresh clean water is introduced to rinse the system. The rinsing step continues until the cleanliness target has been achieved. It is a common practice to over-rinse for extended periods of time since there has been no reliable method for continuously monitoring the quality of the water being discharged and to signal and control the completion of the rinse cycle.

This CIP system is time-consuming and costly, especially since the materials of construction are attacked and damaged by the high temperature and aggressive chemicals often used in the process. Extended periods of time are necessary to thoroughly rinse out the residues of the cleaning agents, and the detection limit of these materials is too high to measure to extinction.

Other multiple-step CIP systems are known in the art. For example, there is a 5-step CIP system, which involves the following steps: pre-rinsing; cleaning; rinsing; cleaning; and final rinsing.

Accordingly, there exists a need to provide a less harsh and more efficient method to monitor and control a CIP system.

WO 94/09357 relates to a process for determining the concentration of an active agent containing a tracer in aqueous or non-aqueous solutions of active agents. The concentration is measured via a determination of its tracer contents. The tracer used is a fluorescent colorant which measures the fluorescent colorant in the solution photo-optically and determines the agent's concentration correspondingly from the measurement obtained. The active agent concentration and any eventual subsequent dosage can be determined reliably, accurately, rapidly, continuously and with few breakdowns.

### SUMMARY OF THE INVENTION

The present invention provides for a method for monitoring a CIP system comprising the steps of:
a. providing a fluorometer;
b. fluorometrically monitoring said CIP system;
c. pre-rinsing said CIP system with potable water;
d. adding a known first amount of fluorescent tracer and a known first amount of chemical to said CIP system or the chemical with fluorescent properties alone to said CIP system;
e. circulating said fluorescent tracer and said chemical for a pre-determined time period or circulating said chemical alone for a pre-determined time period;
f. using said fluorometer to detect the fluorescence of either said chemical, said fluorescent tracer, or both, in said CIP system, wherein said fluorometer produces an output signal proportional to the detected fluorescence;
g. optionally adjusting the dosage amount of said fluorescent tracer and chemical or chemical alone based upon the output signal from said fluorometer;
h. final rinsing said CIP system with potable water until the output signal from said fluorometer can no longer be detected or indicates a predetermined level of said fluorescent tracer, chemical or both;
i. adding a known amount of a second-stage fluorescent tracer to said CIP system; and
j. flushing said CIP system with potable water until the output signal from said fluorometer for said second-stage tracer can no longer be detected or indicates a pre-determined level of said second-stage tracer. The present invention also provides that this method further comprises performing the following steps between steps g. and h.:
   a. rinsing said CIP system with potable water;
   b. adding a known second amount of fluorescent tracer and a known second amount of chemical to said CIP system or the chemical alone to said CIP system;
   c. circulating said fluorescent tracer and said chemical for a pre-determined time period or circulating said chemical alone for a pre-determined time period;
   d. using said fluorometer to detect the fluorescence of either said chemical, said fluorescent tracer, or both, in said CIP system, wherein said fluorometer produces an output signal proportional to the detected fluorescence; and
   e. optionally repeating the preceding steps one or more times.

### DESCRIPTION OF THE INVENTION

Throughout this patent application the following terms have the indicated meanings:
"CIP" means Clean-In-Place.
"GMP" means Good Manufacturing Practices.
"Pre-rinse" means the rinse stage prior to adding chemical to the CIP system.
"Final-rinse" means the rinse stage prior to adding the second stage tracer.
"Second-stage tracer" means a tracer that is added after the Final-Rinse stage of the CIP system.

It is known in the art of fluorescent tracer technology to relate the fluorescent signal of a fluorescent tracer to the amount of fluorescent tracer present. Then by knowing the amount of fluorescent tracer present, the amount of chemical present can be calculated, because a known amount of a fluorescent tracer is always added to a known amount of chemical, thus making the proportional relationship between the fluorescent tracer and the chemical added known. When the chemical has fluorescent properties itself the quantity of chemical can be obtained from its fluorescent signal. Also, a combination of monitoring the fluorescence of the chemical itself and fluorescent tracer is another method for determining the quantity of chemical present.

The fluorometer produces an output signal proportional to the detected fluorescence. Optionally, adjusting the dosage of the fluorescent tracer and chemical or chemical alone is based on the output signal from said fluorescent tracer and/or said chemical detected by the fluorometer.

In one embodiment the fluorescent tracer can be inert and is herein referred to as inert fluorescent tracer. The term "inert," as used herein refers to an inert fluorescent tracer that is not appreciably or significantly affected by any other chemistry in the system, or by the other system parameters such as pH, temperature, ionic strength, redox potential, microbiological activity or biocide concentration. To quantify what is meant by "not appreciably or significantly affected", this statement means that an inert fluorescent compound has no more than a 10% change in its fluorescent signal, under severe conditions in industrial CIP system.

It should be appreciated that a variety of different and suitable inert fluorescent tracers can be utilized in any suitable amount, number and application. In an embodiment, inert fluorescent tracer monitoring of the present invention can be conducted on a singular, intermittent or semi-continuous basis, and preferably the concentration determination of the tracer in the CIP system is conducted on-site to provide a rapid real-time determination.

An inert tracer must be transportable with the water of the CIP system and thus substantially, if not wholly, water-soluble therein at the concentration it is used, under the temperature and pressure conditions specific and unique to the CIP system. In other words, an inert fluorescent tracer displays properties similar to a solute of the CIP system in which it is used. In an embodiment, it is preferred that the inert fluorescent tracer of the present invention meet the following criteria:
1. Be substantially foreign to the chemical species that are normally present in the water of the CIP system in which the inert fluorescent tracer(s) may be used;
2. Be substantially impervious to interference from, or biasing by, the chemical species that are normally present in the water of the CIP system in which the inert tracer(s) may be used;
3. Be compatible with all chemicals added to the water of the CIP system in which the inert fluorescent tracer(s) may be used, and thus in no way reduce the efficacy thereof;
4. Be compatible with all components of its formulation; and
5. Be relatively nontoxic and environmentally safe, not only within the environs of the CIP system in which it may be used, but also upon discharge therefrom.

It should be appreciated that the amount of inert fluorescent tracer to be added to the CIP system that is effective without being grossly excessive can vary with respect to a variety of factors including, without limitation, the monitoring method selected, the extent of background interference associated with the selected monitoring method, the magnitude of the expected inert fluorescent tracer(s) concentration in the CIP system, the monitoring mode (such as, an on-line continuous monitoring mode), and other similar factors. In an embodiment, the amount of tracer added to said CIP system ranges from about 5 ppt to about 1000 ppm, preferably from about 1 ppb to about 50 ppm, more preferably from about 5 ppb to about 50 ppb.

In another embodiment, the fluorescent tracer can be added to a CIP system as a component of a formulation, rather than as a separate component, such as a dry solid or neat far liquid. The fluorescent tracer formulation or product may include an aqueous solution or other substantially homogeneous mixture that disperses with reasonable rapidity in the CIP system to which it is added. In this regard, the fluorescent tracer's concentration may be correlated to the concentration of a product. In another embodiment, the chemical added has an inherent fluorescent property. In another embodiment, the chemical added to the CIP system is tagged with moiety capable of fluorescing.

In another embodiment of the invention, both the chemical and/or fluorescent tracer are circulated in the CIP system for a time period according to GMP.

In another embodiment of the invention, the inert fluorescent tracer can be pyrene tetrasulfonic acid, tetrasodium salt.

In another embodiment, the inert fluorescent tracer is selected from the group consisting of 3,6-acridinediamine, N,N,N',N'-tetramethyl, monohydrochloride; 2-anthracenesulfonic acid sodium salt; 1,5-anthracenedisulfonic acid; 2,6-anthracenedisulfonic acid; 1,8-anthracenedisulfonic acid; anthra[9,1,2-cde]benzo[rst]pentaphene-5,10-diol, 16,17-dimethoxy-,bis(hydrogen sulfate), disodium salt; bathophenanthrolinedisulfonic acid disodium salt; amino 2,5-benzene disulfonic acid; 2-(4-aminophenyl)-6-methylbenzothiazole; 1H-benz[de]isoquinoline-5-sulfonic acid, 6-amino-2,3-dihydro-2-(4-methylphenyl)-1,3-dioxo-, monosodium salt; phenoxazin-5-ium, 1aminocarbonyl)-7-(diethylamino)3,4-dihydroxy-, chloride; benzo[a]phenoxazin-7-ium, 5,9-diamino-, acetate; 4-dibenzofuransulfonic acid; 3-dibenzofuransulfonic acid; 1-ethylquinaldinium iodide; fluorocein; fluorescein, sodium salt; Keyfluor White ST; benzenesulfonic acid, 2,2'-(1,2-ethenediyl)bis[5-[[4-[bis(2-hydroxyethyl)amino]-6-[(4-sulfopheny 1)amino]-1,3,5-triazin-2-yl]amino]-, tetrasodium salt; C.I. Florescent Brightener 230; benzenesulfonic acid, 2,2'-(1,2-ethenediyl)bis[5-[[4-[bis(2-hydroxyethyl)amino]-6-[(4-sulfophenyl)amino]-1,3,5-triazin-2-yl]amino], tetrasodium salt; 9,9'-biacridinium, 10,10'-dimethyl-, dinitrate; 1-deoxy-1-(3,4-dihydro-7,8-dimethyl-2,4-dioxobenzo[g]pteridin-10(2H)-yl)-ribitol; mono-, di-, or tri-sulfonated napthalenes selected from the group consisting of 1,5-naphthalenedisulfonic acid, disodium salt (hydrate); 2-amino-1-naphthalenesulfonic acid; 5-amino-2-naphthalenesulfonic acid; 4amino-3-hydroxy-1-naphthalenesulfonic acid; 6-amino-4-hydroxy-2-naphthalenesulfonic acid; 7-amino-1,3-naphthalenesulfonic acid, potassium salt; 4-amino-5-hydroxy-2,7-naphthalenedisulfonic acid; 5-dimethylamino-1-naphthalenesulfonic acid; 1-amino-4-naphthalene sulfonic acid; 1-amino-7-naphthalene sulfonic acid; and 2,6-naphthalenedicarboxylic acid, dipotassium salt; 3,4,9,10-perylenetetracarboxylic acid; C.I. Fluorescent Brightener 191; C.I. Fluorescent Brightener 200; benzenesulfonic acid, 2,2'-(1,2-ethenediyl)bis[5-(4-phenyl-2H-1,2,3-triazol-2-yl), dipotassium salt; benzenesulfonic acid, 5-(2H-naphtho[1,2-d]triazol-2-yl)-2(2-phenylethenyl)-, sodium salt; 1,3,6,8-pyrenetetrasulfonic acid, tetrasodiun salt; pyranine; quinoline; 3H-phenoxazin-3-one, 7-hydroxy-, 10-oxide; xanthylium, 9-(2,4-dicarboxyphenyl)-3,6-bis(diethylamino)-, chloride, disodium salt; phenazinium, 3,7-diamino-2,8-dimethyl-5-phenyl-, chloride; C.I. Fluorescent Brightener 235; benzenesulfonic acid, 2,2'-(1,2-ethenediyl)bis[5-[[4-[bis(2-hydroxyethyl)amino]-6-[(4-sulfopheny 1)amino]-1,3,5-triazin-2-yl]amino]-, tetrasodium salt; benzenesulfonic acid, 2,2'-(1,2-ethenediyl)bis[5-[[4-[(2-hydroxypropyl)amino]-6-(phenylamino)-1, 3,5-triazin-2-yl]amino]-, disodium salt; xanthylium, 3,6-bis(diethylamino)-9-(2,4-disulfophenyl)-, inner salt, sodium salt; benzenesulfonic acid, 2,2'-(1,2-ethenediyl)bis[5-[[4-[(aminomethyl)(2-hydroxyethyl)amino]-6-(phenylamino)-1,3,5-triazin-2-yl]amino]-, disodiun salt; Tinopol DCS; benzenesulfonic acid, 2,2'-([1,1'-biphenyl]-4,4'-diyldi-2,1-ethenediyl)bis, disodium salt; benzenesulfonic acid, 5-(2H-naphtho[1,2-d]triazol-2-yl)-2-(2-phenylethenyl)-, sodium salt; 7-benzothiazolesulfonic acid, 2,2'-(1-triazene-1,3-diyldi-4,1-phenylene)bis[6-methyl-, disodium salt; and all ammonium, potassium and sodium salts thereof; and all mixtures thereof, wherein said components of said mixtures are selected such that the fluorescent signals of the individual inert fluorescent tracers within the mixture are capable of being detected.

In another embodiment of the invention, the chemical added to the CIP system is selected from the group consisting of: cleaners, sanitizers, or a combination thereof; peracetic acid; biocides; chlorine bleach; chlorine dioxide; glutaraldehyde; and 2,2-dibromo-3-nitrile propionamide; and mixtures thereof.

In another embodiment of the invention, the second stage tracer that is added to the CIP system is selected from the group consisting of: food ingredient solution; sodium benzoate; tannic acid; quinine; and dodecyl benzene sulfonic acid.

In another embodiment, final rinsing of said CIP system with potable water continues until the output signal from the fluorometer indicates a predetermined level of said fluourescent tracer or chemical of less than about 1 ppb.

In another embodiment, final rinsing of said CIP system with potable water continues until the output signal from the fluorometer indicates a predetermined level of said second-stage tracer of less than about 1 ppb.

The following examples are presented to describe preferred embodiments and utilities of the invention and is not meant to limit the invention unless otherwise stated in the claims appended hereto.

### EXAMPLE 1, not according to the invention:

A fluorometer would be set up to measure a certain fluorophor, having a signature excitation and emission wavelength. The fluorometer is included in the CIP system so that any water that is circulating through the CIP system is also circulating through the detection device. When the chemical and fluorescent tracer combination product is injected into the loop, the solution created passes through the fluorometer, and the desired parameters are measured. As soon as the fluorometer is powered it begins measurement of fluorescence of a certain signature wavelength. Finding none, the fluorometer activates a relay that activates a feed pump connected to a container of the combination product. A target setpoint for fluorescence has been programmed into the memory of the instrumentation, and when the setpoint is reached the fluorometer signals the pump to stop. In this way the fluorometer can both monitor and control the dosage of chemical. A datalogger captures the signal from the fluorometer and stores this is in a digital format for later analysis or for use as a step in a computer based program. The system is then rinsed with the fluorescent tracer readings measuring the % of flushing (or alternatively the level of CIP chemical still remaining) that has occurred and when the system has reached to prescribed level of flushing (or alternatively the level of CIP chemical treatment has been sufficiently removed).

### EXAMPLE 2, not according to the invention:

A fluorometer would be set up to measure a certain fluorophor, having a signature excitation and emission wavelength. The fluorometer is included in the CIP system so that any water that is circulating through the CIP system is also circulating through the detection device. When the chemical and fluorescent tracer combination product is injected into the loop, the solution created passes through the fluorometer, and the desired parameters are measured. As soon as the fluorometer is powered it begins measurement of fluorescence of a certain signature wavelength. Finding none, the fluorometer activates a relay that activates a feed pump connected to a container of the combination product. A target setpoint for fluorescence has been programmed into the memory of the instrumentation, and when the setpoint is reached the fluorometer signals the pump to stop. In this way the fluorometer can both monitor and control the dosage of chemical. A datalogger captures the signal from the fluorometer and stores this is in a digital format for later analysis or for use as a step in a computer based program. The system is then rinsed with the fluorescent tracer readings measuring the % of flushing (or alternatively the level of CIP chemical still remaining) that has occurred and when the system has reached to prescribed level of flushing (or alternatively the level of CIP chemical treatment has been sufficiently removed). The system cleaning with fluorescent tracer combination chemical being reapplied and the flushing step is then repeated. Monitoring and control of the CIP chemical treatment dosage and flushing of the system based on the fluorescent tracer signal is also repeated as above described.

### EXAMPLE 3 (according to the invention):

A fluorometer would be set up to measure a certain fluorophor, having a signature excitation and emission wavelength. The fluorometer is included in the CIP system so that any water that is circulating through the CIP system is also circulating through the detection device. When the chemical and fluorescent tracer combination product is injected into the loop, the solution created passes through the fluorometer, and the desired parameters are measured. As soon as the fluorometer is powered it begins measurement of fluorescence of a certain signature wavelength. Finding none, the fluorometer activates a relay that activates a feed pump connected to a container of the combination product. A target setpoint for fluorescence has been programmed into the memory of the instrumentation, and when the setpoint is reached the fluorometer signals the pump to stop. In this way the fluorometer can both monitor and control the dosage of chemical. A datalogger captures the signal from the fluorometer and stores this is in a digital format for later analysis or for use as a step in a computer based program. The system is then rinsed with the fluorescent tracer readings measuring the % of flushing (or alternatively the level of CIP chemical still remaining) that has occurred. Once the system has reached an initial prescribed % level of flushing (and an initial reduction in the dosage of CIP chemical remaining), then a second addition of fluorescent tracer only is added and further flushing of the system occurs. This allows the measurement of the level of flushing to be even more sensitively determined and determination of the level of remaining CIP chemical to be measured to an even lower dosage.

For example, 100 ppm of CIP chemical and 1 ppm of fluorescent tracer may have been added initially. During the initial flushing procedure, the fluorometer measures that fluorescent tracer dosage has decreased to 0.001 ppm (which corresponds to 99.9% flushing of the system and reduction of CIP chemical to 0.1 ppm). Then a second addition of fluorescent tracer only is made to attain a 1 ppm dosage of fluorescent tracer. The system is further flushed so that a final dosage of 0.001 ppm of fluorescent tracer is obtained. This would correspond to 99.9999% flushing of the system and a reduction in the original dosage of CIP chemical to 0.0001 ppm or (0.1 ppb). This step of further adding of fluorescent tracer only, after the original fluorescent tracer combination chemical has been added can be repeated as many times as needed in order to reach the final % level of flushing of the system. When the system has reached to prescribed level of flushing (or alternatively the level of CIP chemical treatment has been sufficiently removed). This further adding of a fluorescent tracer can utilize the same fluorescent tracer as was present in the fluorescent tracer combination chemical or can be a different fluorescent tracer (depending on whether government regulations and whether natural or government approved substances are only allowed in the final rinsing step).

## Claims

1. A method for monitoring a CIP (Clean-In-Place) system comprising the steps of:
a. providing a fluorometer;
b. fluorometrically monitoring said CIP system;
c. pre-rinsing said CIP system with potable water;
d. adding a known first amount of fluorescent tracer and a known first amount of chemical to said CIP system or the chemical with fluorescent properties alone to said CIP system;
e. circulating said fluorescent tracer and said chemical for a pre-determined time period or circulating said chemical alone for a pre-determined time period;
f. using said fluorometer to detect the fluorescence of either said chemical, said fluorescent tracer, or both, in said CIP system, wherein said fluorometer produces an output signal proportional to the detected fluorescence;
g. optionally adjusting the dosage amount of said fluorescent tracer and chemical or chemical alone based upon the output signal from said fluorometer;
h. final rinsing said CIP system with potable water until the output signal from said fluorometer can no longer be detected or indicates a predetermined level of said fluorescent tracer, chemical or both:
i. adding a known amount of a second-stage fluorescent tracer to said CIP system; and
j. flushing said CIP system with potable water until the output signal from said fluorometer for said second-stage tracer can no longer be detected or indicates a pre-determined level of said second-stage tracer.

2. The method of claim 1 further comprising performing the following steps between steps g. and h.:
a. rinsing said CIP system with potable water;
b. adding a known second amount of fluorescent tracer and a known second amount of chemical to said CIP system or the chemical alone to said CIP system;
c. circulating said fluorescent tracer and said chemical for a pre-determined time period or circulating said chemical alone for a pre-determined time period;
d. using said fluorometer to detect the fluorescence of either said chemical, said fluorescent tracer, or both, in said CIP system, wherein said fluorometer produces an output signal proportional to the detected fluorescence; and
e. optionally repeating the preceding steps one or more times.

3. The method of claim 1 wherein said fluorescent tracer is an inert fluorescent tracer.

4. The method of claim 3 wherein the concentration of said inert fluorescent tracer fed into the CIP system is from about 5 ppt to about 1000 ppm.

5. The method of claim 3 wherein the concentration of said inert fluorescent tracer fed into the CIP system is from about 1 ppb to about 50 ppm.

6. The method of claim 3 wherein the concentration of said inert fluorescent tracer fed into the CIP system is from about 5ppb to about 50 ppb.

7. The method of claim 3 wherein the inert fluorescent tracer is selected from the group consisting of 3,6-acridinediamine, N,N,N',N'-tetramethyl, monohydrochloride; 2-anthracenesulfonic acid sodium salt; 1,5-anthracenedisulfonic acid; 2,6-anthracenedisulfonic acid; 1,8-anthracenedisulfonic acid; anthra[9,1,2-cde]benzo[rst]pentaphene-5,10-diol, 16,17-dimethoxy-,bis(hydrogen sulfate), disodium salt; bathophenanthrolinedisulfonic acid disodium salt; amino 2,5-benzene disulfonic acid; 2-(4-aminophenyl)-6-methylbenzothiazole; 1 H-benz[de]isoquinoline-5-sulfonic acid, 6-amino-2,3-dihydro-2-(4-methylphenyl)-1,3-dioxo-, monosodium salt; phenoxazin-5-ium, 1aminocarbonyl)-7-(diethylamino)3,4-dihydroxy-, chloride; benzo[a]phenoxazin-7-ium, 5,9-diamino-, acetate; 4-dibenzofuransulfonic acid; 3-dibenzofuransulfonic acid; 1-ethylquinaldinium iodide; fluorocein; fluorescein, sodium salt; Keyfluor White ST; benzenesulfonic acid, 2,2'-(1,2-ethenediyl)bis[5-[[4-[bis(2-hydroxyethyl)amino]-6-[(4-sulfophenyl)amino]-1,3,5-triazin-2-yl]amino]-, tetrasodium salt; C.I. Florescent Brightener 230; benzenesulfonic acid, 2,2'-(1,2-ethenediyl)bis[5-[[4-[bis(2-hydroxyethyl)amino]-6-[(4-sulfophenyl)amino]-1,3,5-triazin-2-yl]amino], tetrasodium salt; 9,9'-biacridinium, 10,10'-dimethyl-, dinitrate; 1-deoxy-1-(3,4-dihydro-7,8-dimethyl-2,4-dioxobenzo[g]pteridin-10(2H)-yl)-ribitol; mono-, di-, or tri-sulfonated napthalenes selected from the group consisting of 1,5-naphthalenedisulfonic acid, disodium salt (hydrate); 2-amino-1-naphthalenesulfonic acid; 5-amino-2-naphthalenesulfonic acid; 4amino-3-hydroxy-1-naphthalenesulfonic acid; 6-amino-4-hydroxy-2-naphthalenesulfonic acid; 7-amino-1,3-naphthalenesulfonic acid, potassium salt; 4-amino-5-hydroxy-2,7-naphthalenedisulfonic acid; 5-dimethylamino-1-naphthalenesulfonic acid; 1-amino-4-naphthalene sulfonic acid; 1-amino-7-naphthalene sulfonic acid; and 2,6-naphthalenedicarboxylic acid, dipotassium salt; 3,4,9,10-perylenetetracarboxylic acid; C.I. Fluorescent Brightener 191; C.I. Fluorescent Brightener 200; benzenesulfonic acid, 2,2'-(1,2-ethenediyl)bis[5-(4-phenyl-2H-1,2,3-triazol-2-yl), dipotassium salt; benzenesulfonic acid, 5-(2H-naphtho[1,2-d]triazol-2-yl)-2(2-phenylethenyl)-, sodium salt; 1,3,6,8-pyrenetetrasulfonic acid, tetrasodiun salt; pyranine; quinoline; 3H-phenoxazin-3-one, 7-hydroxy-, 10-oxide; xanthylium, 9-(2,4-dicarboxyphenyl)-3,6-bis(diethylamino)-, chloride, disodium salt; phenazinium, 3,7-diamino-2,8-dimethyl-5-phenyl-, chloride; C.I. Fluorescent Brightener 235; benzenesulfonic acid, 2,2'-(1,2-ethenediyl)bis[5-[[4-[bis(2-hydroxyethyl)amino]-6-[(4-sulfopheny 1)amino]-1,3,5-triazin-2-yl]amino]-, tetrasodium salt; benzenesulfonic acid, 2,2'-(1,2-ethenediyl)bis[5-[[4-[(2-hydroxypropyl)amino]-6-(phenylamino)-1, 3,5-triazin-2-yl]amino]-, disodium salt; xanthylium, 3,6-bis(diethylamino)-9-(2,4-disulfophenyl)-, inner salt, sodium salt; benzenesulfonic acid, 2,2'-(1,2-ethenediyl)bis[5-[[4-[(aminomethyl)(2-hydroxyethyl)amino]-6-(phenylamino)-1,3,5-triazin-2-yl ]amino]-, disodiun salt; Tinopol DCS; benzenesulfonic acid, 2,2'-([1,1'-biphenyl]-4,4'-diyldi-2,1-ethenediyl)bis, disodium salt; benzenesulfonic acid, 5-(2H-naphtho[1,2-d]triazol-2-yl)-2-(2-phenylethenyl)-, sodium salt; 7-benzothiazolesulfonic acid, 2,2'-(1-triazene-1,3-diyldi-4,1-phenylene)bis[6-methyl-, disodium salt; and all ammonium, potassium and sodium salts thereof; and all mixtures thereof, wherein said components of said mixtures are selected such that the fluorescent signals of the individual inert fluorescent tracers within the mixture are capable of being detected.

8. The method of claim 1 wherein said pre-determined level of said fluorescent tracer or chemical is less than about 1 ppb.

9. The method of claim 1 wherein said pre-determined level of said second-stage tracer is less than about 1 ppb.

10. The method of claim 1 wherein a formulation of fluorescent tracer and chemical are added to said CIP system.

11. The method of claim 1 wherein said chemical added to said CIP system has a tagged fluorescent moiety.

12. The method of claim 3 wherein said inert fluorescent tracer is pyrene tetrasulfonic acid, tetrasodium salt.

13. The method of claim 1 wherein said second stage tracer is selected from the group consisting of: food ingredient solution; sodium benzoate; tannic acid; quinine; and dodecyl benzene sulfonic acid.

14. The method of claim 1 wherein said chemical is selected from the group consisting of: cleaners, sanitizers, or a combination thereof; biocides; peracetic acid; hydroxide; chlorine bleach; chlorine dioxide; glutaraldehyde; and 2,2-dibromo-3-nitrile propionamide; and mixtures thereof.

15. The method of claim 1 wherein said pre-determined time period is a time period according to GMP (Good Manufacturing Practices).

## Patentansprüche

1. Verfahren zur Überwachung eines *in*-*situ*-Reinigungsystems, umfassend die Schritte:
a. Bereitstellen eines Fluorometers;
b. fluorometrisches Überwachen des *in*-*situ*-Reinigungsystems;
c. Vorspülen des *in*-*situ*-Reinigungsystems mit Trinkwasser;
d. Zugeben einer bekannten, ersten Menge eines fluoreszierenden Tracers und einer bekannten, ersten Menge einer Chemikalie zum *in-situ*-Reinigungsystem, oder nur der Chemikalie mit Fluoreszenz-Eigenschaften zum *in*-*situ*-Reinigungsystem;
e. Zirkulieren des fluoreszierenden Tracers und der Chemikalie für einen vorgegebenen Zeitraum, oder nur der Chemikalie für einen vorgegebenen Zeitraum;
f. Verwenden des Fluorometers, um die Fluoreszenz entweder der Chemikalie, des fluoreszierenden Tracers oder beider im *in-situ-*Reinigungssystem zu detektieren, wobei das Fluorometer ein Ausgabesignal erzeugt, welches proportional zur detektierten Fluoreszenz ist;
g. optional das Einstellen der Dosiermenge des fluoreszierenden Tracers und der Chemikalie, oder nur der Chemikalie, auf Basis des Ausgabesignals des Fluorometers;
h. abschließendes Spülen des *in*-*situ*-Reinigungssystems mit Trinkwasser, bis das Ausgabesignal des Fluorometers nicht länger detektiert werden kann oder eine vorgegebene Konzentration des fluoreszierenden Tracers, der Chemikalie oder beider anzeigt;
i. Zugeben einer bekannten Menge eines fluoreszierenden Tracers für die zweite Phase zum *in*-*situ*-Reinigungssystem; und
j. Durchspülen des *in*-*situ*-Reinigungssystems mit Trinkwasser, bis das Ausgabesignal des Fluorometers für den fluoreszierenden Tracer der zweiten Phase nicht länger detektiert werden kann oder eine vorgegebene Konzentration des Tracers der zweiten Phase anzeigt.

2. Verfahren nach Anspruch 1, ferner umfassend das Durchführen der folgenden Schritte zwischen den Schritten g. und h.:
a. Spülen des *in*-*situ*-Reinigungssystems mit Trinkwasser;
b. Zugeben einer bekannten, zweiten Menge an fluoreszierendem Tracer und einer bekannten, zweiten Menge an Chemikalie zum *in-situ-*Reinigungssystem, oder nur der Chemikalie zum *in*-*situ*-Reinigungssystem;
c. Zirkulieren des fluoreszierenden Tracers und der Chemikalie für einen vorgegebenen Zeitraum, oder Zirkulieren nur der Chemikalie für einen vorgegebenen Zeitraum;
d. Verwenden des Fluorometers, um die Fluoreszenz entweder der Chemikalie, des fluoreszierenden Tracers oder beider im *in-situ-*Reinigungssystem zu detektieren, wobei das Fluorometer ein Ausgabesignal erzeugt, welches proportional ist zur detektierten Fluoreszenz; und
e. optional das ein- oder mehrmalige Wiederholen der vorhergehenden Schritte.

3. Verfahren nach Anspruch 1, wobei der fluoreszierende Tracer ein inerter fluoreszierender Tracer ist.

4. Verfahren nach Anspruch 3, wobei die Konzentration des inerten, dem *in-situ*-Reinigungssystem zugegebenen fluoreszierenden Tracers von etwa 5 ppt bis etwa 1000 ppm reicht.

5. Verfahren nach Anspruch 3, wobei die Konzentration des inerten, dem *in-situ*-Reinigungssystem zugegebenen fluoreszierenden Tracers von etwa 1 ppb bis etwa 50 ppm reicht.

6. Verfahren nach Anspruch 3, wobei die Konzentration des inerten, dem *in-situ*-Reinigungssystem zugegebenen fluoreszierenden Tracers von etwa 5 ppb bis etwa 50 ppb reicht.

7. Verfahren nach Anspruch 3, wobei der inerte fluoreszierende Tracer ausgewählt ist aus der Gruppe bestehend aus N,N,N',N'-Tetramethyl-3,6-acridindiaminmonohydrochlorid; 2-Anthracensulfonsäure-Natriumsalz; 1,5-Anthracendisulfonsäure; 2,6-Anthracendisulfonsäure; 1,8-Anthracendisulfonsäure; Anthra[9,1,2-cde]benzo[rst]pentaphen-5,10-diol-16,17-dimethoxy-bis(hydrogensulfat)-Dinatriumsalz; Bathophenanthrolindisulfonsäure-Dinatriumsalz; Amino-2,5-benzoldisulfonsäure; 2-(4-Aminophenyl)-6-methylbenzothiazol; 6-Amino-2,3-dihydro-2-(4-methylphenyl)-1,3-dioxo-1H-benz[de]isochinolin-5-sulfonsäure-Mononatriumsalz; 1-(Aminocarbonyl)-7-(diethylamino)-3,4-dihydroxyphenoxazin-5-iumchlorid; 5,9-Diaminobenzo[a]phenoxazin-7-iumacetat; 4-Dibenzofuransulfonsäure; 3-Dibenzofuransulfonsäure; 1-Ethylchinaldiniumiodid; Fluorescein; Fluorescein-Natriumsalz; Keyfluor White ST; 2,2'-(1,2-Ethendiyl)bis[5-[[4-[bis(2-hydroxyethyl)amino]-6-[(4-sulfophenyl)amino]-1,3,5-triazin-2-yl]amino]benzolsulfonsäure-Tetranatriumsalz; C.I. Fluorescent Brightener 230; 2,2'-(1,2-Ethendiyl)bis[5-[[4-[bis(2-hydroxyethyl)amino]-[(4-sulfophenyl)amino]-1,3,5-triazin-2-yl]amino)benzolsulfonsäure-Tetranatriumsalz; 10,10'-Dimethyl-9,9'-biacridiniumdinitrat; 1-Desoxy-1-(3,4-dihydro-7,8-dimethyl-2,4-dioxobenzo[g]-pteridin-10(2H)-yl)-ribit; mono-, di- oder trisulfonierte Naphthaline, ausgewählt aus der Gruppe bestehend aus 1,5-Naphthalindisulfonsäure-Dinatriumsalz (Hydrat); 2-Amino-1-naphthalinsulfonsäure; 5-Amino-2-naphthalinsulfonsäure; 4-Amino-3-hydroxy-1-naphthalinsulfonsäure; 6-Amino-4-hydroxy-2-naphthalinsulfonsäure; 7-Amino-1,3-naphthalindisulfonsäure-Kaliumsalz; 4-Amino-5-hydroxy-2,7-naphthalindisulfonsäure; 5-Dimethylamino-1-naphthalinsulfonsäure; 1-Amino-4-naphthalinsulfonsäure; 1-Amino-7-naphthalinsulfonsäure und 2,6-Naphthalindicarbocarbonsäure-Dikaliumsalz; 3,4,9,10-Perylentetracarbonsäure; Fluorescent Brightener 191; Fluorescent Brightener 200; 2,2'-(1,2-Ethendiyl)bis[5-(4-phenyl-2H-1,2,3-triazol-2-yl)benzolsulfonsäure-Dikaliumsalz; 5-(2H-Naphtho[1,2-d]triazol-2-yl)-2-(2-phenylethenyl)benzolsulfonsäure-Natriumsalz; 1,3,6,8-Pyrentetrasulfonsäure-Tetranatriumsalz; Pyranin; Chinolin; 3H-Phenoxazin-3-on-7-hydroxy-10-oxid; 9-(2,4-Dicarboxyphenyl)-3,6-bis(diethylamino)xanthyliumchlorid-Dinatriumsalz; 3,7-Diamino-2,8-dimethyl-5-phenyl-phenaziniumchlorid; Fluorescent Brightener 235; 2,2'-(1,2-Ethendiyl)bis[5-[[4-[bis(2-hydroxyethyl)-amino]-6-[(4-sulfophenyl)amino]-1,3,5-15-triazin-2-yl]amino]-benzolsulfonsäure-Tetranatriumsalz; 2,2'-(1,2-Ethendiyl)bis[5-[[4-[(2-hydroxypropyl)amino]-6-(phenylamino)-1,3,5-triazin-2-yl]amino]benzolsulfonsäure-Dinatriumsalz; 3,6-Bis(diethylamino)-9-(2,4-disulfophenyl)xanthylium-Zwitterion-Natriumsalz; 2,2'-(1,2-Ethendiyl)bis[5-[[4-[(aminomethyl)(2-hydroxyethyl)amino]-6-(phenylamino)-1,3,5-triazin-2-yl]amino]benzolsulfonsäure-Dinatriumsalz; Tinopol DCS; 2,2'-([1,1'-Biphenyl]-4,4'-diyldi-2,1-ethendiyl)bisbenzolsulfonsäure-Dinatriumsalz ; 5-(2H-Naphtho[1,2-d]triazol-2-yl)-2-(2-phenylethenyl)-benzolsulfonsäure-Natriumsalz; 2,2'-(1-Triazen-1,3-diyldi-4,1-phenylen)bis-[6-methyl-7-benzothiazolsulfonsäure-Dinatriumsalz und alle Ammonium-, Kalium- und Natriumsalze davon; sowie alle Gemische davon, wobei die genannten Bestandteile der Gemische derartig ausgewählt sind, dass die Fluoreszenz-Signale der einzelnen inerten fluoreszierenden Tracer im Gemisch detektiert werden können.

8. Verfahren nach Anspruch 1, wobei die vorgegebene Konzentration des fluoreszierenden Tracers oder der Chemikalie kleiner ist als ungefähr 1 ppb.

9. Verfahren nach Anspruch 1, wobei die vorgegebene Konzentration des Tracers der zweiten Phase kleiner ist als ungefähr 1 ppb.

10. Verfahren nach Anspruch 1, wobei eine Formulierung aus fluoreszierendem Tracer und Chemikalie zum *in*-*situ*-Reinigungssystem gegeben wird

11. Verfahren nach Anspruch 1, wobei die zum *in*-*situ*-Reinigungssystem zugegebene Chemikalie einen markierten fluoreszierenden Rest aufweist.

12. Verfahren nach Anspruch 3, wobei der inerte fluoreszierende Tracer Pyrentetrasulfonsäure-Tetranatriumsalz ist.

13. Verfahren nach Anspruch 1, wobei der Tracer der zweiten Phase ausgewählt ist aus der Gruppe bestehend aus: einer Lösung von Nahrungsmittelbestandteilen; Natriumbenzoat; Gerbsäure; Chinin und Dodecylbenzolsulfonsäure.

14. Verfahren nach Anspruch 1, wobei die Chemikalie ausgewählt ist aus der Gruppe bestehend aus: Reinigern, Desinfektionsmitteln oder einer Kombination davon; Bioziden; Peressigsäure; Hydroxid; Chlorbleiche; Chlordioxid; Glutaraldehyd und 2,2-Dibrom-3-nitrilpropionamid; sowie Gemische davon.

15. Verfahren nach Anspruch 1, wobei der vorgegebene Zeitraum ein Zeitraum ist gemäß GMP (*Good Manufacturing Practice,* Gute Herstellungspraxis).

## Revendications

1. Procédé de surveillance d'un système de nettoyage en place (NEP) comprenant les étapes suivantes :
a. la fourniture d'un fluoromètre ;
b. la surveillance fluorométrique dudit système NEP ;
c. le pré-rinçage du système NEP avec de l'eau potable ;
d. l'ajout d'une première quantité connue d'un traceur fluorescent et d'une première quantité connue d'un produit chimique audit système NEP ou le produit chimique avec des propriétés fluorescentes seul audit système NEP ;
e. la circulation dudit traceur fluorescent et dudit produit chimique pendant un laps de temps prédéfini ou la circulation dudit produit chimique seul pendant un laps de temps prédéfini ;
f. l'utilisation dudit fluoromètre pour détecter la fluorescence soit dudit produit chimique, soit dudit traceur fluorescent, ou les deux, dans ledit système NEP, dans lequel ledit fluoromètre produit un signal de sortie proportionnel à la fluorescence détectée ;
g. l'ajustement éventuel de la quantité de dosage desdits traceur fluorométrique et produit chimique ou dudit produit chimique seul sur la base du signal de sortie en provenance dudit fluoromètre ;
h. le rinçage final dudit système NET avec de l'eau potable jusqu'à ce que le signal de sortie en provenance dudit fluoromètre ne puisse plus être détecté ou indique un niveau prédéfini dudit traceur fluorescent, dudit produit chimique ou des deux ;
i. l'ajout d'une quantité connue d'un traceur fluorescent de seconde phase audit système NEP ; et
j. la purge dudit système NEP avec de l'eau potable jusqu'à ce que le signal de sortie en provenance dudit fluoromètre pour ledit traceur de seconde phase ne puisse plus être détecté ou indique un niveau prédéfini dudit traceur de seconde phase.

2. Procédé selon la revendication 1, comprenant en outre la mise en oeuvre des étapes suivantes entre les étapes g. et h. :
a. le rinçage dudit système NEP avec de l'eau potable ;
b. l'ajout d'une seconde quantité connue de traceur fluorescent et d'une seconde quantité connue de produit chimique audit système NEP ou le produit chimique seul audit système NEP ;
c. la circulation dudit traceur fluorescent et dudit produit chimique pendant un laps de temps prédéfini ou la circulation dudit produit chimique seul pendant un laps de temps prédéfini ;
d. l'utilisation dudit fluoromètre pour détecter la fluorescence soit dudit produit chimique, soit dudit traceur fluorescent, ou les deux, dans ledit système NEP, dans lequel ledit fluoromètre produit un signal de sortie proportionnel à la fluorescence détectée ; et
e. la répétition éventuelle des étapes précédentes une ou plusieurs fois.

3. Procédé selon la revendication 1, dans lequel ledit traceur fluorescent est un traceur fluorescent inerte.

4. Procédé selon la revendication 3, dans lequel la concentration dudit traceur fluorescent inerte introduit dans le système NEP est d'environ 5 ppt à environ 1 000 ppm.

5. Procédé selon la revendication 3, dans lequel la concentration dudit traceur fluorescent inerte introduit dans le système NEP est d'environ 1 ppb à environ 50 ppm.

6. Procédé selon la revendication 3, dans lequel la concentration dudit traceur fluorescent inerte introduit dans le système NEP est d'environ 5 ppb à environ 50 ppb.

7. Procédé selon la revendication 3, dans lequel le traceur fluorescent inerte est choisi parmi le groupe constitué du 3,6-acridinediamine, N,N,N',N'-tétraméthyle, monohydrochlorate ; du sel de sodium de l'acide 2-anthracènesulfonique ; de l'acide 1,5-anthracènedisulfonique ; de l'acide 2,6-anthracènedisulfonique ; de l'acide 1-8-anthracènedisulfonique ; du sel disodique de l'anthra[9,1,2-cde]benzo[rst]pentaphène-5,10-diol, 16,17-diméthoxy-,bis(sulfate d'hydrogène) ; du sel disodique de l'acide bathophénanthrolinedisulfonique ; de l'acide disulfonique d'amino 2,5-benzène ; du 2-(4-aminophényl)-6-méthylbenzothiazole ; de l'acide 1H-benz[de]isoquinoline-5-sulfonique, du sel monosodique du 6-amino-2,3-dihydro-2-(4-méthylphényl)-1,3-dioxo- ; du phénoxazin-5-ium , du chlorure de 1-(aminocarbonyl)-7-(diéthylamino)-3,4-dihydroxy- ; du benzo[a]phénoxazin-7-ium , de l'acétate de 5,9-diamino-; de l'acide 4-dibenzofuransulfonique ; de l'acide 3-dibenzofuransulfonique ; de l'iodure de 1-éthylquinaldinium ; de la fluorescéine ; du sel de sodium de fluorescéine ; du Keyfluor White ST ; de l'acide benzènesulfonique, du sel tétrasodique de 2,2'-(1,2-éthènediyl)bis[5-[[4-[bis(2-hydroxyéthyl)amino]-6-[(4-sulfophényl)amino]-1,3,5-triazin-2-yl]amino]- ; du C.I. Florescent Brightener 230 ; de l'acide benzènesulfonique, du sel tétrasodique de 2,2'-(1,2-éthènediyl)bis[5-[[4-[bis(2-hydroxyéthyl)amino]-6-[(4-sulfophényl)amino]-1,3,5-triazin-2-yl]amino) ; du 9,9'-biacridinium, du dinitrate de 10,10'-diméthyl- ; du 1-déoxy-1-(3,4-dihydro-7,8-diméthyl-2,4-dioxobenzo[g]ptéridin-10(2H)-yl)-ribitol ; de naphtalènes mono-, di- ou trisulfonés choisis parmi le groupe constitué du sel sodique (hydrate) de l'acide 1,5-naphtalènedisulfonique ; de l'acide 2-amino-1-naphtalènesulfonique ; de l'acide 5-amino-2-naphtalènesulfonique ; de l'acide 4-amino-3-hydroxy-1-naphtalènesulfonique ; de l'acide 6-amino-4-hydroxy-2-naphtalènesulfonique ; du sel de potassium de l'acide 7-amino-1,3-naphtalènesulfonique ; de l'acide 4-amino-5-hydroxy-2,7-naphtalènedisulfonique ; de l'acide 5-diméthylamino-1-naphtalènesulfonique ; de l'acide 1-amino-4-naphtalènesulfonique ; de l'acide 1-amino-7-naphtalènesulfonique ; et du sel de dipotassium de l'acide 2,6-naphtalènedicarboxylique ; de l'acide 3,4,9,10-pérylènetétracarboxylique ; du C.I. Fluorescent Brightener 191 ; du C.I. Fluorescent Brightener 200 ; de l'acide benzènesulfonique, du sel de dipotassium du 2,2'-(1,2-éthènediyl)bis[5-(4-phényl-2H-1,2,3-triazol-2-yl) ; de l'acide benzènesulfonique, du sel de sodium de 5-(2H-naphto[1,2-d]triazol-2-yl)-2(2-phényléthényl)-, ; du sel tétrasodique de l'acide de 1,3,6,8-pyrènetétrasulfonique ; de la pyranine ; de la quinoline ; du 3H-phénoxazin-3-one, du 7-hydroxy-, de 10-oxyde ; du xanthylium, du sel disodique du chlorure de 9-(2,4-dicarboxyphényl)-3,6-bis(diéthylamino)- ; du phénazinium, du chlorure de 3,7-diamino- 2,8-diméthyl-5-phényl- ; du C.I. Fluorescent Brightener 235 ; de l'acide benzènesulfonique, du sel tétrasodique de 2,2'-(1,2-éthènediyl)bis[5-[[4-[(2-hydroxypropyl)amino]-6-(phénylamino)-1,3,5-triazin-2-yl]amino]- ; du xanthylium, du zwitterion, du sel de sodium de 3,6-bis(diéthylamino)-9-(2,4-disulfophényl) ; de l'acide benzènesulfonique, du sel disodique de 2,2'-(1,2-éthènediyl)bis[5-[[4-[(aminométhyl)(2-hydroxyéthyl)amino]- 6-(phénylamino)-1,3,5-triazin-2-yl]amino]- ; du Tinopol DCS ; de l'acide benzènesulfonique, du sel disodique de 2,2'-([1,1'-biphényl]-4-4'-diyldi-2,1-éthènediyl)bis ; de l'acide benzènesulfonique, du sel de sodium de 5-(2H-naphto[1,2-d]triazol-2-yl)-2-(2-phényléthényl)- ; de l'acide 7-benzothiazolesulfonique, du sel disodique de 2,2'-(1-triazène-1,3-diyldi-4,1-phénylène)bis[6-méthyl-; et de tous les sels d'ammonium, de potassium et de sodium de ceux-ci ; et de tous les mélanges de ceux-ci, dans lequel lesdits constituants desdits mélanges sont choisis de sorte que les signaux fluorescents des traceurs fluorescents inertes individuels à l'intérieur du mélange soient aptes à être détectés.

8. Procédé selon la revendication 1, dans lequel ledit niveau prédéfini dudit traceur fluorescent ou dudit produit chimique est inférieur à environ 1 ppb.

9. Procédé selon la revendication 1, dans lequel ledit niveau prédéfini dudit traceur de seconde phase est inférieur à environ 1 ppb.

10. Procédé selon la revendication 1, dans lequel une formulation de traceur fluorescent et de produit chimique est ajoutée audit système NEP.

11. Procédé selon la revendication 1, dans lequel ledit produit chimique ajouté audit système NEP a une fraction fluorescente marquée.

12. Procédé selon la revendication 3, dans lequel ledit traceur fluorescent inerte est du sel tétrasodique de l'acide tétrasulfonique de pyrène.

13. Procédé selon la revendication 1, dans lequel ledit traceur de seconde phase est choisi parmi le groupe constitué : d'une solution d'ingrédient alimentaire ; du benzoate de sodium ; de l'acide tannique ; de la quinine ; et de l'acide sulfonique de dodécylbenzène.

14. Procédé selon la revendication 1, dans lequel ledit produit chimique est choisi parmi le groupe constitué : des agents nettoyants, des désinfectants, ou d'une combinaison de ceux-ci ; des biocides ; de l'acide peracétique ; de l'hydroxyde ; d'un produit à blanchir au chlore ; du dioxyde de chlore ; du glutaraldéhyde ; et du 2,2-dibromo-3-nitrile propionamide ; et des mélanges de ceux-ci.

15. Procédé selon la revendication 1, dans lequel ledit laps de temps prédéfini est un laps de temps selon la bonne pratique de fabrication (BPF).
